# EUROPEAN PATENT APPLICATION

(11) **EP 1 106 603 A2**
(43) Date of publication of application: **13.06.2001**
(21) Application number: 00126448.0
(22) Date of filing: 06.12.2000
(51) Int. Cl.: C07C 317/08, C12Q 1/68

(54) **DNA chip and reactive solid carrier**

(30) Priority: 06.12.1999 JP 34615799; 22.12.1999 JP 36379599; 24.03.2000 JP 2000084373
(71) Applicant: FUJI PHOTO FILM CO., LTD., Kanagawa-ken, 250-0123 (JP)
(72) Inventor: Shinoki, Hiroshi, c/o Fuji Photo Film Co.,Ltd., Asaka-shi, Saitama 351-8585 (JP); Makino, Yoshihiko, c/o Fuji Photo Film Co.,Ltd., Asaka-shi, Saitama 351-8585 (JP); Takeshita, Yumiko, c/o Fuji Photo Film Co.,Ltd., Asaka-shi, Saitama 351-8585 (JP); Sudo, Yukio, c/o Fuji Photo Film Co.,Ltd., Asaka-shi, Saitama 351-8585 (JP); Seshimoto, Osamu, c/o Fuji Photo Film Co.,Ltd., Asaka-shi, Saitama 351-8585 (JP); Yamanouchi, Junichi, c/o Fuji Photo Film Co.,Ltd., Minami-ashigara-shi, Kanagawa 250-0123 (JP)
(74) Representative: Albrecht, Thomas, Dr.

(57) **Abstract**

A reactive solid carrier favorably employable for manufacturing DNA chip is composed of a solid carrier and a plurality of vinylsulfonyl groups or their reactive precursors each of which is fixed onto a surface of the solid carrier by covalent bonding via a linking group, and a method for producing a DNA chip is performed by bringing the reactive solid carrier into contact with nucleotide derivatives or their analogues having a reactive group which is reactive with the vinylsulfonyl group or reactive precursor fixed to the solid carrier.

## Description

### FIELD OF THE INVENTION

This invention relates to a solid carrier to which nucleotide derivatives or their analogues (e.g., oligonucleotides, polynucleotides, and peptide-nucleotides) are attached, which is generally named DNA chip and which is favorably employable for detecting, with high sensitivity, complementary nucleic acid fragments.

### BACKGROUND OF THE INVENTION

Detection of a nucleic acid fragment is generally performed using a probe oligonucleotide which is complementary to the nucleic acid fragment to be detected, by way of hybridization. The probe oligonucleotide is generally fixed onto a solid carrier (e.g., solid substrate) to produce a so-called DNA chip. In the detection procedures, a nucleic acid fragment in a sample liquid is provided with a fluorescent label or a radioisotope label, and then the sample liquid is brought into contact with the probe oligonucleotide of the DNA chip. If the labelled nucleic acid fragment in the sample liquid is complementary to the probe oligonucleotide, the labelled nucleic acid fragment is combined with the probe oligonucleotide by hybridization. The labelled nucleic acid fragment fixed to the DNA chip by hybridization with the probe oligonucleotide is then detected by an appropriate detection method such as fluorometry or autoradiography. The DNA chip is widely employed in the gene technology, for instance, for detecting a complementary nucleic acid fragment and sequencing a nucleic acid.

The DNA chip can be utilized to efficiently detect a large number of complementary nucleic acid fragments in a small amount of a sample liquid almost simultaneously.

Detection of nucleic acid fragment using an electrochemical label is also known (Japanese Patent Provisional Publication No. 9-288080, and a preprint of the 57th Analytical Chemistry Conference pp. 137-138 (1996)).

P.E. Nielsen et al., Science, 254, 1497-1500(1991) and P.E. Nielsen et al., Biochemistry, 36, pp.5072-5077 (1997) describe PNA (Peptide Nucleic Acid or Polyamide Nucleic Acid) which has no negative charge and functions in the same manner as DNA fragment does. PNA has a polyamide skeleton of N-(2-aminoethyl)glycine units and has neither glucose units nor phosphate groups.

Since PNA is electrically neutral and is not charged in the absence of an electrolytic salt, PNA is able to hybridize with a complementary nucleic acid fragment to form a hybrid which is more stable than the hybrid structure given by a probe oligonucleotide and its complementary nucleic acid fragment (Preprint of the 74th Spring Conference of Japan Chemical Society, pp. 1287, reported by Naomi Sugimoto).

Japanese Patent Provisional Publication No.11-332595 describes a PNA probe fixed onto a solid carrier at its one end and a detection method utilizing the PNA probe. The PNA probe is fixed onto the solid carrier by the known combination of avidin and biotin.

The aforementioned P.E. Nielsen et al., Science, 254, 1497-1500(1991) also describes a PNA probe labelled with an isotope element and a detection method of a complementary nucleic acid fragment.

Since the PNA probe shows no electric repulsion to a target nucleic acid fragment in a sample liquid, an improved high detection sensitivity is expected.

At present, two methods are known for preparing a DNA chip having a solid carrier and oligonucleotides or polynucleotides fixed onto the carrier. One preparation method comprises preparing oligonucleotides or polynucleotides step by step on the carrier. This method is named "on-chip method". A typical on-chip method is described in Foder, S.P.A., Science, 251, page 767 (1991).

Another preparation method comprises fixing separately prepared oligonucleotides or polynucleotides onto a solid carrier. Various methods are known for various oligonucleotides and polynucleotides.

In the case that the complementary nucleotide derivatives (which are synthesized using mRNA as mold) or PCR products (which are DNA fragments prepared by multiplying cDNA by PCR method), an aqueous solution of the prepared DNA fragment is spotted onto a solid carrier having a poly-cationic coat in a DNA chip-preparing device to fix the DNA fragment to the carrier via electrostatic bonding, and then blocking a free surface of the polycationic coat.

In the case that the oligonucleotides are synthetically prepared and have a functional group, an aqueous solution of the synthetic oligonucleotides is spotted onto an activated or reactive solid carrier to produce covalent bonding between the oligonucleotides and the carrier surface. See Lamture, J.B., et al., Nucl. Acids Res., 22, 2121-2125, 1994, and Guo, Z., et al., Nucl. Acids Res., 22, 5456-5465, 1994. Generally, the oligonucleotides are covalently bonded to the surface activated carrier via linking groups.

Also known is a process comprising the steps of aligning small polyacrylamide gels on a glass plate and fixing synthetic oligonucleotides onto the glass plate by making a covalent bond between the polyacrylamide and the oligonucleotide (Yershov, G., et al., Proc. Natl. Acad. Sci. USA, 94, 4913(1996)). Sosnowski, R.G., et al., Proc. Natl. Acad. Sci. USA, 94, 1119-1123 (1997) discloses a process comprising the steps of an array of -microelectrodes on a silica chip, forming on the microelectrode a streptoavidin-comprising agarose layer, and attaching biotin-modified DNA fragment to the agarose layer by positively charging the agarose layer. Schena, M., et al., Proc. Natl. Acadl Sci. USA, 93, 10614-10619 (1996) teaches a process comprising the steps of preparing a suspension of an amino group-modified PCR product in SSC (i.e., standard sodium chloride-citric acid buffer solution), spotting the suspension onto a slide glass, incubating the spotted glass slide, treating the incubated slide glass with sodium borohydride, and heating thus treated slide glass.

As is explained above, most of the known methods of fixing separately prepared DNA fragments onto a solid carrier utilize the electrostatic bonding or the covalent bonding such as described above.

In any DNA chips having separately prepared oligonucleotide probes on its solid carrier, the oligonucleotide probes should be firmly fixed onto the carrier, so that the hybridization can proceed smoothly between the fixed oligonucleotide probes and target DNA fragments complementary to the fixed oligonucleotide probes.

United States Patent No. 5,387,505 describes a method of separating a target DNA fragment by binding target DNA fragments labelled with a biotin molecule with a substrate having abidin molecules.

United States Patent No. 5,094,962 discloses a detection tool for a ligand-receptor assay in which receptor molecules are bonded to a porous polymer particle having a reactive group.

### SUMMARY OF THE INVENTION

It is an object of the present invention to provide a reactive solid carrier which is favorably employable for preparing a solid carrier having nucleotide derivatives or their analogues on its surface.

It is another object of the invention to provide a solid carrier having nucleotide derivative probes or their analogue probes on its surface, which is. favorably employable as so-called DNA chip for fixing and detecting oligonucleotides or polynucleotides such as DNA fragments by way of hybridization.

The present invention resides in a reactive solid carrier comprising a solid carrier and a plurality of vinylsulfonyl groups or their reactive precursors each of which is fixed onto a surface of the solid carrier by covalent bonding via a linking group.

In the reactive solid carrier of the invention, the vinylsulfonyl group or its reactive precursor linked to the linking group is preferably represented by the following formula:

- L - SO₂ - X

in which L represents a linking group, and X represents a group of -CR¹=CR²R³ or -CHR^{l}-CR²R³Y wherein each of R¹, R² and R³ independently is a hydrogen atom, an alkyl group having 1 to 6 carbon atoms, an aryl group having 6 to 20 carbon atoms, or an aralkyl group having 7 to 26 carbon atoms in which its alkyl group has 1 to 6 carbon atoms; Y represents a halogen atom, -S0₂R¹¹, -OCOR¹², -OSO₃M, or a quaternary pyridinium group; R¹¹ is a hydrogen atom, an alkyl group having 1 to 6 carbon atoms, an aryl group having 6 to 20 carbon atoms, or an aralkyl group having 7 to 26 carbon atoms in which its alkyl group has 1 to 6 carbon atoms; R¹² is an alkyl group having 1 to 6 carbon atoms, or a halogenated alkyl group having 1 to 6 carbon atoms; and M is a hydrogen atom, an alkali metal atom, or an ammonium group.

The reactive solid carrier of the invention is preferably produced by bringing a solid carrier having reactive groups on its surface into contact with disulfone compounds having the following formula:

X¹ - SO₂ - L² - SO₂ - X²

in which L² represents a linking group, and each of X¹ and X² represents a group of -CR¹=CR²R³ or -CHR¹-CR²R³Y wherein each of R¹, R² and R³ independently is a hydrogen atom, an alkyl group having 1 to 6 carbon atoms, an aryl group having 6 to 20 carbon atoms, or an aralkyl group having 7 to 26 carbon atoms in which its alkyl group has 1 to 6 carbon atoms; Y represents a halogen atom, -SO₂R¹¹, -OCOR¹², -OSO₃M, or a quaternary pyridinium group; R¹¹ is a hydrogen atom, an alkyl group having 1 to 6 carbon atoms, an aryl group having 6 to 20 carbon atoms, or an aralkyl group having 7 to 26 carbon atoms in which its alkyl group has 1 to 6 carbon atoms; R¹² is an alkyl group having 1 to 6 carbon atoms, or a halogenated alkyl group having 1 to 6 carbon atoms; and M is a hydrogen atom, an alkali metal atom, or an ammonium group.

The DNA fragment-detection tool of the invention, that is, a solid carrier having probes of nucleotide derivatives or their analogues fixed onto its surface is preferably manufactured by bringing a reactive solid car rier comprising a solid carrier and a plurality of vinylsulfonyl groups or their reactive precursors each of which is fixed onto a surface of the solid carrier by covalent bonding into contact with nucleotide derivatives or their analogues having a reactive group which is reactive with the vinylsulfonyl group or reactive precursor. Representative examples of the nucleotide derivatives or the analogues are oligonucleotides, polynucleotides, and peptide nucleic acids (i.e., PNA) .

The detection method of the invention for oligonucleotides or polynucleotides such as DNA fragments can be performed by bringing the solid carrier having probes of nucleotide derivatives or their analogues fixed onto its surface into contact with oligonucleotides or polynucleotides (such as target DNA fragments) which are complementary to the probes of nucleotide derivatives or their analogues fixed onto the surface of the solid carrier in the presence of an aqueous solvent, so as to combine the complementary oligonucleotides or polynucleotides with the nucleotide derivatives or their analogues.

The reactive solid carrier of the invention also can be produced by bringing a solid carrier having reactive groups on its surface into contact with bifunctional compounds having the following formula:

X¹ - SO₂ - L² - X³

in which L² represents a linking group, X¹ represents a group of -CR¹=CR²R³ or -CHR¹-CR²R³Y wherein each of R¹, R² and R³ independently is a hydrogen atom, an alkyl group having 1 to 6 carbon atoms, an aryl group having 6 to 20 carbon atoms, or an aralkyl group having 7 to 26 carbon atoms in which its alkyl group has 1 to 6 carbon atoms; Y represents a halogen atom, -S0₂R¹¹, -OCOR¹², -OSO₃M, or a quaternary pyridinium group; R¹¹ is a hydrogen atom, an alkyl group having 1 to 6 carbon atoms, an aryl group having 6 to 20 carbon atoms, or an aralkyl group having 7 to 26 carbon atoms in which its alkyl group has 1 to 6 carbon atoms; R¹² is an alkyl group having 1 to 6 carbon atoms, or a halogenated alkyl group having 1 to 6 carbon atoms; and M is a hydrogen atom, an alkali metal atom, or an ammonium group, and X³ represents a maleimide group, a halogen atom, an isocyanate group, a thioisocyanate group, a succimidoxy group, an aldehyde group, or a carboxyl group.

The reactive solid carrier of the invention may comprise a solid carrier and a plurality of vinylsulfonyl groups or their reactive precursors each of which is fixed onto a surface of the solid carrier by covalent bonding via hydrophilic polymer chains. In this reactive solid carrier, the vinylsulfonyl group or its reactive precursor linked to the linking group is preferably represented by the following formula:

- L - SO₂ - X

in which L represents a hydrophilic polymer chain, and X represents a group of -CR¹=CR²R³ or -CHR¹-CR²R³Y wherein each of R¹, R² and R³ independently is a hydrogen atom, an alkyl group having 1 to 6 carbon atoms, an aryl group having 6 to 20 carbon atoms, or an aralkyl group having 7 to 26 carbon atoms in which its alkyl group has 1 to 6 carbon atoms; Y represents a halogen atom, -SO₂R¹¹, -OCOR¹², -OSO₃M, or a quaternary pyridinium group; R¹¹ is a hydrogen atom, an alkyl group having 1 to 6 carbon atoms, an aryl group having 6 to 20 carbon atoms, or an aralkyl group having 7 to 26 carbon atoms in which its alkyl group has 1 to 6 carbon atoms; R¹² is an alkyl group having 1 to 6 carbon atoms, or a halogenated alkyl group having 1 to 6 carbon atoms; and M is a hydrogen atom, an alkali metal atom, or an ammonium group.

Accordingly, the DNA fragment-detection tool of the invention, that is, a solid carrier having probes of nucleotide derivatives or their analogues fixed onto its surface can be produced by bringing a reactive solid carrier comprising a solid carrier and a plurality of vinylsulfonyl groups or their reactive precursors each of which is fixed onto a surface of the solid carrier by covalent bonding via a hydrophilic polymer chain into contact with nucleotide derivatives or their analogues having a reactive group which is reactive with the vinylsulfonyl group or reactive precursor.

The reactive solid carrier of the invention also may comprise a solid carrier and a plurality of vinylsulfonyl groups or their reactive precursors a number of which are linked to a polymer chain which is fixed onto a surface of the solid carrier by covalent bonding at a number of sites which number is smaller than the number of vinylsulfonyl groups or their reactive precursors linked to the polymer chain.

In the above-mentioned reactive solid carrier, the vinylsulfonyl group or its reactive precursor linked to the polymer chain is preferably represented by the following formula:

- L -(- SO₂ - X)_{K}

in which L represents a polymer chain, X represents a group of -CR¹=CR²R³ or -CHR¹-CR²R³Y wherein each of R¹, R² and R³ independently is a hydrogen atom, an alkyl group having 1 to 6 carbon atoms, an aryl group having 6 to 20 carbon atoms, or an aralkyl group having 7 to 26 carbon atoms in which its alkyl group has 1 to 6 carbon atoms; Y represents a halogen atom, -SO₂R¹¹, -OCOR¹², -OSO₃M, or a quaternary pyridinium group; R11 is a hydrogen atom, an alkyl group having 1 to 6 carbon atoms, an aryl group having 6 to 20 carbon atoms, or an aralkyl group having 7 to 26 carbon atoms in which its alkyl group has 1 to 6 carbon atoms; R¹² is an alkyl group having 1 to 6 carbon atoms, or a halogenated alkyl group having 1 to 6 carbon atoms; and M is a hydrogen atom, an alkali metal atom, or an ammonium group, and k is an integer of 2 or more.

The solid carrier having nucleotide derivatives or their analogues fixed onto its surface, that is so-called DNA chip, can be manufactured by bringing a reactive solid carrier comprising a solid carrier and a plurality of vinylsulfonyl groups or their reactive precursors a number of which are linked to a polymer chain which is fixed onto a surface of the solid carrier by covalent bonding at a number of sites which number is smaller than the number of vinylsulfonyl groups or their reactive precursors linked to the polymer chain into contact with nucleotide derivatives or their analogues having a reactive group which is reactive with the vinylsulfonyl group or reactive precursor.

### BRIEF DESCRIPTION OF DRAWINGS

Fig. 1 schematically illustrates a representative oligonucleotide-fixed solid carrier and its production processes according to the invention.

Fig. 2 schematically illustrates a process for fixing an oligonucleotide probe onto a solid carrier according to the invention.

Fig. 3 schematically illustrates a process for fixing an oligonucleotide probe onto a solid carrier using a hydrophilic polymer which is also according to the invention.

Fig. 4 schematically illustrates a process for fixing an oligonucleotide probe onto a solid carrier using a multi-functional polymer chain which is also according to the invention.

### DETAILED DESCRIPTION OF THE INVENTION

### [Solid Carrier]

The solid carrier can be any of known solid carriers or their equivalent materials, for instance, a glass plate, a resin plate, a metal plate, a glass plate covered with polymer coat, a glass plate covered with metal coat, and a resin plate covered with metal coat. Also employable is a SPR (surface plasmon resonance) sensor plate which is described in Japanese Patent Provisional Publication No. 11-332595. CCD is also employable as described in Nucleic Acids Research, 1994, Vol.22, No.11, 2124-2125.

The solid carrier should have a plurality of reactive groups such as amino, aldehyde, epoxy, carboxyl, mercapto, or hydroxyl. Particularly preferred is an amino group. The amino group can be placed on the carrier by processing the carrier with a silane-coupling agent or coating an amine group-containing polymer (i.e., poly-cationic polymer) such as poly-L-lysine, polyethyleneimine, or polyalkylamine. When the silane-coupling agent is employed, the coupling agent is fixed to the carrier via covalent-bonding. When the amine group-containing polymer is employed, the polymer is fixed to the carrier by electrostatic bonding. Naturally, the covalent-bonding is preferred from the viewpoint of stable and reliable fixation.

Examples of the silane-coupling agents include γaminopropyltriethoxysilane, N-β (aminoethyl)-γ-aminopropyltrimethoxysilane, and N-β (aminoethyl) -γ-aminopropylmethyldimethoxysilane. Most preferred is γ-aminopropyltriethoxysilane.

A combination of processing a solid carrier with a silane-coupling agent in combination with coating with a poly-cationic polymer is also employable.

### [Reactive solid carrier]

The reactive solid carrier of the present invention comprises a solid carrier and a plurality of vinylsulfonyl groups or their reactive precursors each of which is fixed onto a surface of the solid carrier by covalent bonding via a linking group.

The vinylsulfonyl group or its reactive precursor linked to the linking group is preferably represented by the following formula:

- L - SO₂ - X

In the formula, X represents a group of -CR^{l}=CR²R³ or -CHR¹-CR²R³Y. Each of R¹, R² and R³ independently is a hydrogen atom, an alkyl group having 1 to 6 carbon atoms such as methyl, ethyl, n-propyl, isopropyl, n-butyl, or n-hexyl, an aryl group having 6 to 20 carbon atoms such as phenyl or naphthyl, or an aralkyl group having 7 to 26 carbon atoms in which its alkyl group has 1 to 6 carbon atoms such as benzyl or phenethyl.

Y is a group which. can be substituted with a nucleophilic reagent such as -OH, -OR°, -SH, NH₃, or NH₂R° (R° can be an alkyl group) or which is released by a base in the form of HY- Examples of the groups of Y include a halogen atom, -SO₂R¹¹, -OCOR¹², -OSO₃M, or a quaternary pyridinium group. R¹¹ is a hydrogen atom, an alkyl group having 1 to 6 carbon atoms, an aryl group having 6 to 20 carbon atoms, or an aralkyl group having 7 to 26 carbon atoms in which its alkyl group has 1 to 6 carbon atoms. R¹² is an alkyl group having 1 to 6 carbon atoms, or a halogenated alkyl group having 1 to 6 carbon atoms. M is a hydrogen atom, an alkali metal atom, or an ammonium. group which may have one or more substituents.

Representative examples of the groups of X are illustrated below:

(X1) -CH=CH₂ (X2) -Ch₂CH₂-Br (X3) -CH₂CH₂-Cl

(X4) -CH₂CH₂-OSO₂CH₃ (X5) -CH₂CH₂-OSO₂C₆H₅ (X6) -CH₂CH₂-OSO₂C₆H₄-CH₃

(X7) - CH₂CH₂OSO₃Na (X8) -CH₂CH₂-OSO₃K

(X9) -CH₂CH₂-OCOCH₃ (X10) -CH₂CH₂-OCOCF₃ (X11) -CH₂CH₂-OCOCHCI₂

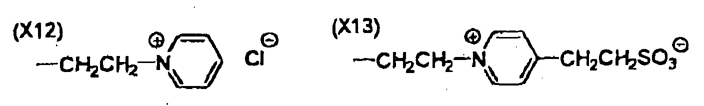

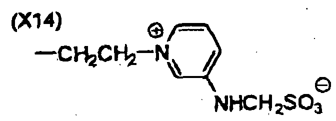

Preferred are the groups of (X1), (X2), (X3), (X4) , (X7), (X8), (X13) and (Xl4). More preferred are the groups of (X1) and (X2). Most preferred is the group of (X1), that is, a vinylsulfonyl group.

In the above-mentioned formula, L represents a linking group of divalence or multiple valence, which links the group of -SO₂-X to the solid carrier or another linking group attached to the solid carrier. L may be a single bond or a hydrophilic polymer chain. L may be linked to two or more -SO₂-X groups. Examples of linking groups for L include an alkylene group having 1 to 6 carbon atoms, an alicyclic group having 3 to 16 carbon atoms, an arylene group having 6 to 20 carbon atoms, a heterocyclic group having 2 to 20 carbon atoms and 1 to 3 hetero_ atoms such as N, S, or P, a divalent group such as. -O-, -S-, -SO-, -SO₂-, -SO₃-, -NR¹¹- (R¹¹ is a hydrogen atom, an alkyl group having 1-15 carbon atoms, preferably, 1-6 carbon atoms such as methyl or ethyl, an aryl group having 6-20 carbon atoms, or an aralkyl group of 7 to 21 carbon atoms having an alkyl group of 1 to 6 carbon atoms), or -CO-. These linking groups can be present alone. However, the linking groups can be used in combination. Therefore, L can be -NR¹¹-, -SONR¹¹-, -CONR¹¹-, - NR¹¹COO-, and -NR¹¹CONR¹¹- If two or more of R¹¹ are present in one linking group, these R¹¹ can be combined to form a ring. The alkyl group, aryl group, and aralkyl group for R¹¹ can have one or more substituents such as a hydroxyl group, an alkoxy group having 1 to 6 carbon atoms, an alkenyl group having 1 to 6 carbon atoms, a carbamoyl group having 2 to 7 carbon atoms, an alkyl group having 1 to 6 carbon atoms, an aralkyl group having 7 to 16 carbon atoms, an aryl group having 6 to 20 carbon atoms, a sulfamoyl group (or its salt with Na, K, or other cation), a carboxyl group (or its salt with Na, K, or other cation), a halogen atom, an alkenylene group having 1 to 6 carbon atoms, an arylene group having 6 to 20 carbon atoms, a sulfonyl group, or combinations of these groups and atoms.

Preferred examples of the L of the formula are illustrated below (in which "a" is an integer of 1 to 6, preferably 1 or 2, and "b" is an integer of 0 to 6, preferably 2 or 3) :

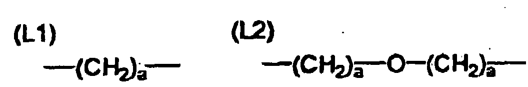

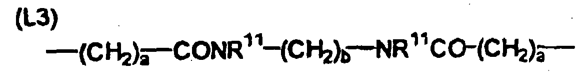

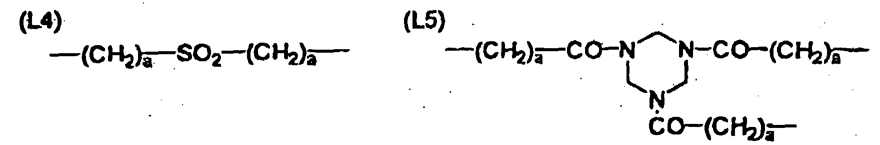

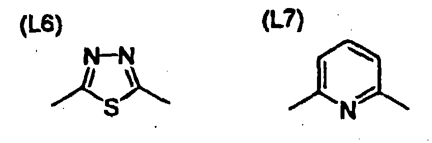

The alkylene group of the above-illustrated divalent or trivalent groups can have a substituent group represented by -SO₂CH=CH₂.

As for the group of "-L-SO₂-X", the following groups also can be mentioned:

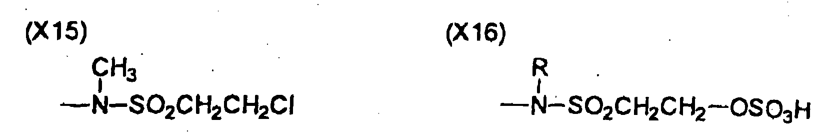

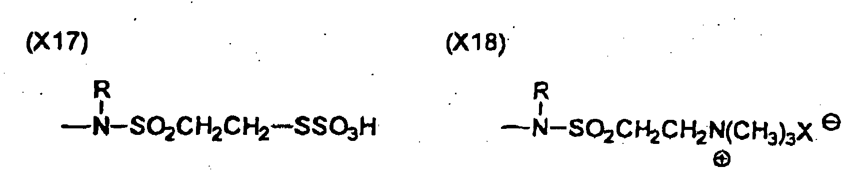

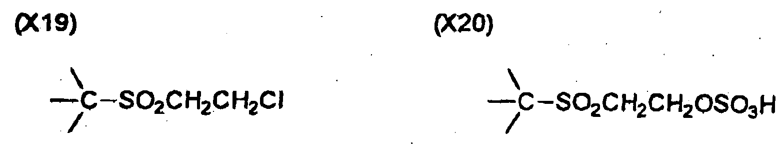

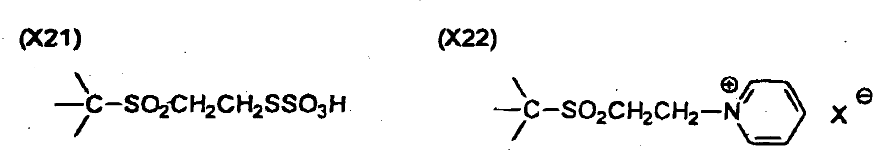

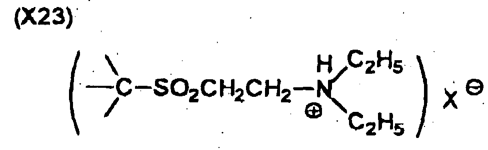

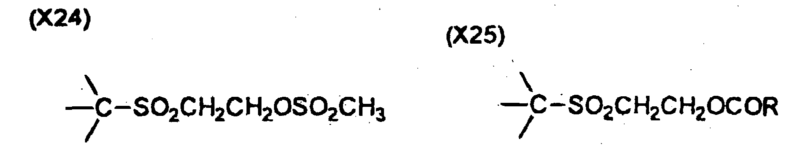

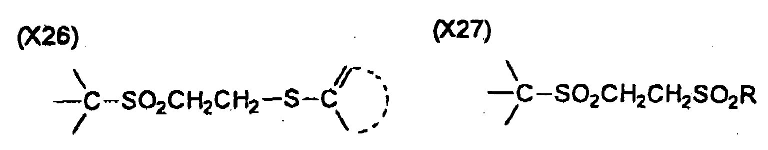

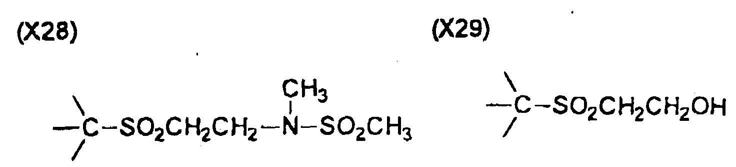

The reactive solid carrier of the invention is preferably produced by bringing a solid carrier having reactive groups on its surface into contact with disulfone compounds having the following formula:

X¹ - SO₂ - L² - SO₂ - X²

Each of X¹ and X² is one of groups of -CR¹=CR²R³ and -CHR¹-CR²R³Y which are already described for the group of X. L² is a linking group such as one already described for L.

The disulfone compound can be brought into contact with the solid carrier in the presence of an aqueous medium to prepare a reactive solid carrier of the invention.

Representative examples of the disulfone compounds are illustrated below.

(S1) H₂C=CH-SO₂-CH₂-SO₂-CH=CH₂

(S2) H₂C=CH-SO₂-CH₂OCH₂-SO₂-CH=CH₂

(S3) H₂C=CH-SO₂-CH₂CH₂CH₂-SO₂-CH=CH₂

(S4) H₂C=CH-SO₂-CH₂CH(OH)CH₂-SO₂-CH=CH₂

(S5) H₂C=CH-SO₂-CH₂CONHCH₂CH₂NHCOCH₂-SO₂-CH=CH₂

(S6) H₂C=CH-SO₂-CH₂CONHCH₂CH₂CH₂NHCOCH₂-SO₂-CH=CH₂

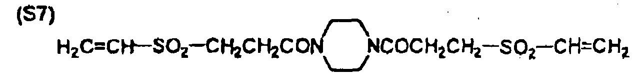

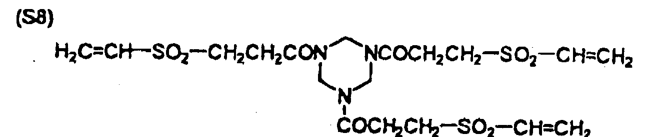

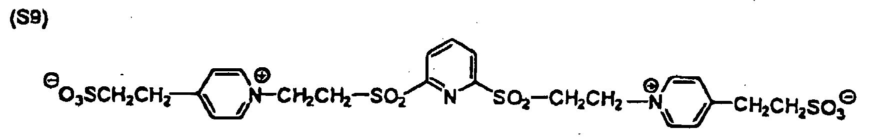

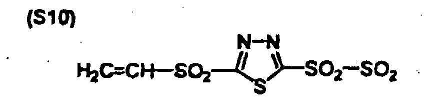

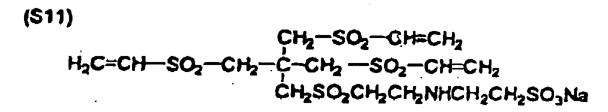

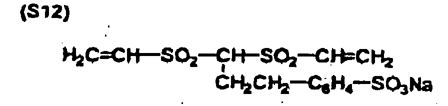

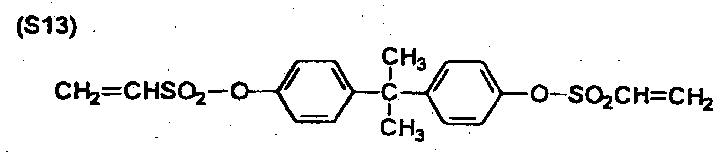

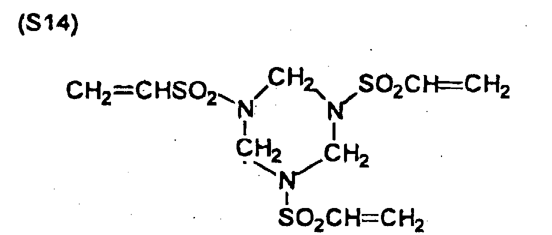

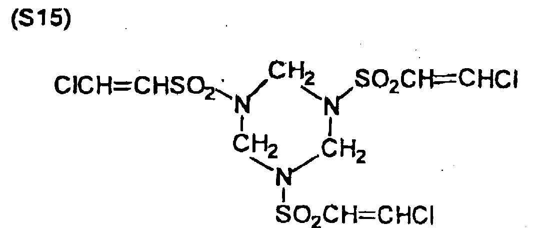

Most preferred is l, 2-bis (vinylsulfonylamide) ethane which corresponds to (S1).

The disulfone compounds can be synthesized in the manners described in Japanese Patent Publications No. 47-2429 and No 50-35807, and Japanese Patent Provisional Publications No. 49-24435, No. 53-41551 and No. 59-18944.

The reactive solid carrier of the invention can be also prepared by bringing a solid carrier having reactive groups on its surface into contact with the following bifunctional compounds:

X¹ - SO₂ - L² - X3

In the formula, X¹ is one of groups of -CR¹=CR²R³ and -CHR¹-CR²R³Y which are already described for the group of X. L² is a linking group such as one already described for L.

X³ is a maleimide group, a halogen atom, an isocyanate group, a thioisocyanate group, a succimidoxy group, an aldehyde group, or a carboxyl group.

The bifunctional compound of the above-identified formula can be brought into contact with the solid carrier in the presence of an aqueous medium to prepare a reactive solid carrier of the invention.

### [Nucleotide Derivative]

The nucleotide derivatives or their analogues to be fixed to the solid carrier can be oligonucleotides, polynucleotides, or peptide-nucleotides. The nucleotide derivatives may be DNA fragments. The nucleotide derivative may be polynucleotide such as cDNA, a portion of cDNA, or EST. The polynucleotide is favorably employed for studying gene expression. Otherwise, nucleotide derivatives to be fixed onto the solid carrier may be oligonucleotides, which are favorably employed for studying variations and polymorphism of gene. The oligonucleotide to be fixed onto the solid carrier preferably is one of 3 to 50-mers, more preferably 10 to 25 mers. The oligonucleotide and polynucleotide can have one or more substituent groups or cross-linking groups, provided that the attachment of these groups does not impart adverse influence to the function of the oligonucleotide and polynucleotide. For instance, LNA (locked nucleic acid) which is described in J. Am. Chem. Soc., 1998, 120, 13252-13253, can be employed.

The nucleotide derivative or its analogue to be employed in the invention should have at its one terminal or its vicinity a reactive group which can react with the vinylsulfonyl group or its reactive precursor. The reactive group can be a group of amino, carboxyl, acyl, or carbamoyl. Most preferred is an amino group.

The reactive group can be attached to the nucleotide derivative or its analogue via a linking group. The linking group preferably is an alkylene group or an Nalkylamino-alkylene group. Preferred are a hexylene group and an N-methylamino-hexylene group.

### [Mechanism of Fixation of Nucleotide derivatives]

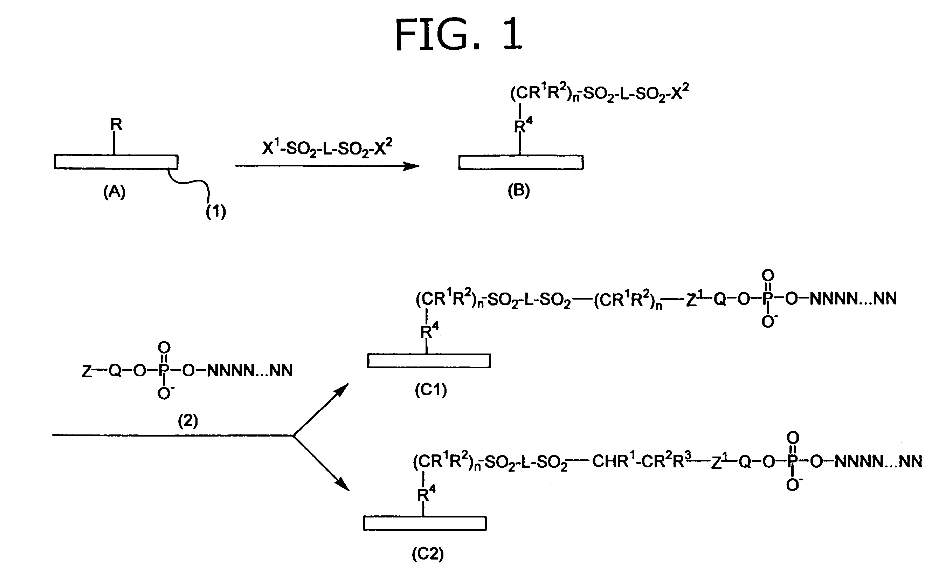

In Fig. 1, a typical process for fixing a nucleotide derivative (typically, oligonucleotide) to a solid carrier according to the invention is schematically illustrated.

In Fig. 1, (1) indicates a solid carrier, R is a reactive group attached to the solid carrier, (A) is a solid carrier having the reactive group. Each of X¹ and X² is a group represented by -CR¹=CHR², and L is a linking group. B is a reactive solid carrier to which a vinylsulfonyl-containing reactive group is attached via a linking group of R⁴. -NNNN...NN- is an oligonucleotide moiety, Q is a linking group, and Z is a covalent bonding site.

In the step (2), the reactive solid carrier (B) is brought into contact with the oligonucleotide to give a DNA chip (C1) or (C2), in which (C1) is produced when X² is a group represented by -CR¹=CHR² and (C2) is produced when X² is a group represented by -CR¹=CR²R³.

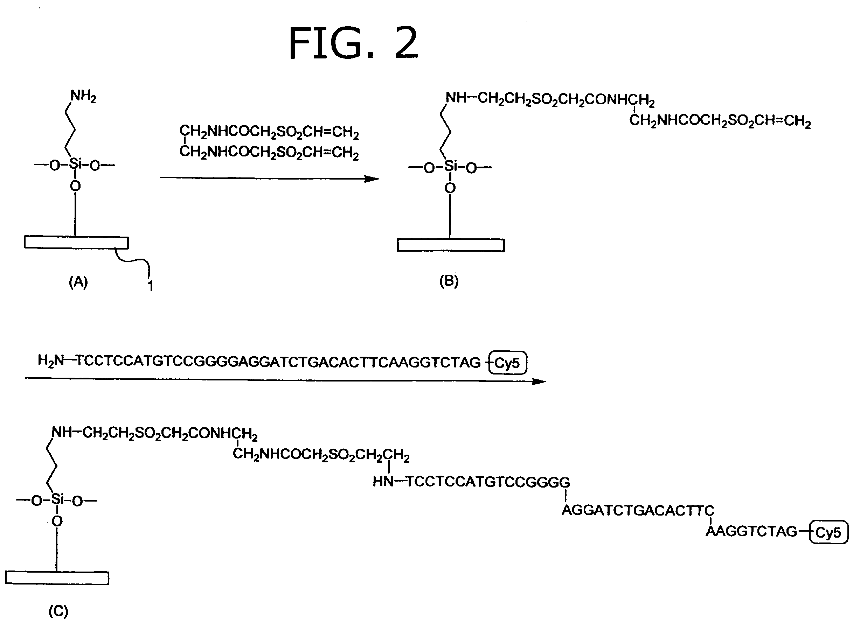

Fig. 2 illustrates the fixation of an oligonucleotide having a fluorescent label (Cy5) to a solid carrier (A) using a silane coupling agent and 1,2-bis(vinylsulfonylamide)ethane. The reactive solid carrier is indicated by (B) and the resulting DNA chip is indicated by (C)

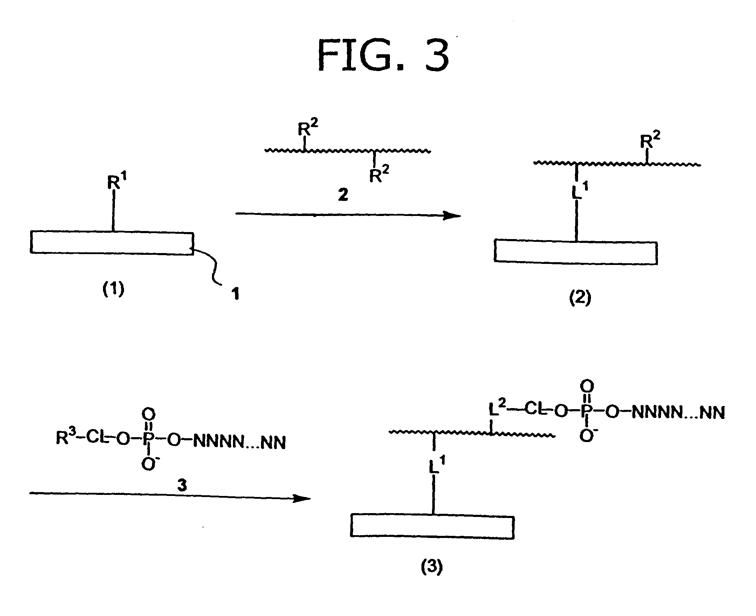

Fig. 3 illustrates the fixation of an oligonucleotide to a solid carrier (1) having a reactive group (R¹) on its surface. The solid carrier is brought into contact with a hydrophilic polymer having two or more vinylsulfonyl groups (R²) in the step denoted by 2. In the resulting reactive solid carrier (2), the polymer chain is attached to the solid carrier via a linking group L¹. In the step denoted by 3, the reactive solid carrier (2) is brought into contact with an oligonucleotide to which is attached a reactive group R³ via a cross-linker (CL). The reactive group R² and the reactive group R³ are reacted with each other to form a linking group L².

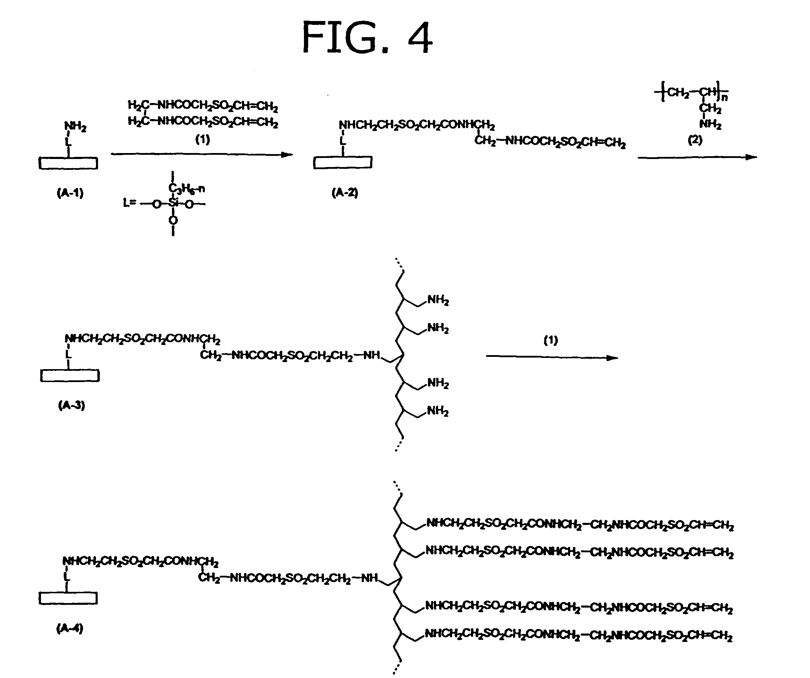

Fig. 4 illustrates the production of a reactive solid carrier onto which a plurality of vinylsulfonyl groups are attached using a multi-functional polymer chain.

### [Procedure of Fixation}

The nucleotide derivatives (or their analogues) to be fixed on the solid carrier are dissolved or dispersed in an aqueous solution. Generally, the aqueous solution is once placed on a plastic plate having 96 or 384 wells, and then spotted onto a solid carrier using a spotting means.

In order to keep the spotted aqueous solution from evaporating, it is preferred to add a high boiling-point compound to the aqueous solution containing nucleotide derivatives. The high boiling-point compound should be soluble in an aqueous medium, should not disturb hybridization procedure, and preferably has an appropriate viscosity. Examples of the high boiling-point compounds include glycerol, ethylene glycol, dimethylsulfoxide, and a hydrophilic polymer having a low molecular weight (typically, in the range of 10³ to 10⁶) such as polyacrylamide, polyethylene glycol, or poly(sodium acrylate). The high boiling-point compound preferably is glycerol or ethylene glycol. The high boiling-point compound is preferably incorporated into an aqueous nucleotide derivative solution in an amount of 0.1 to 2 vol.%, particularly 0.5 to 1 vol.%. Otherwise, the spotted aqueous solution is preferably kept at under the conditions of a high humidity (such as 90%RH or more) and an ordinary temperature (25 to 50°C) .

The aqueous solution is spotted onto the solid carrier under the condition that each drop of the solution generally has a volume of 100 pL to 1 µL, preferably 1 to 100 nL. The nucleotide derivatives preferably spotted onto the solid carrier are in an amount (number) of 10² to 10⁵/cm². In terms of mol., 1 to 10⁻¹⁵ moles are spotted. In terms of weight, several ng or less of nucleotide derivatives are spotted. The spotting of the aqueous solution is made onto the solid carrier to form several dots having almost the same shape and size. It is important to prepare these dots to have the same shape and size, if the hybridization is quantitatively analyzed. Several dots are formed separately from each other with a distance of 1.5 mm or less, preferably 100 to 300 µm. One dot preferably has a diameter of 50 to 300 µm.

After the aqueous solution is spotted on the solid carrier, the spotted solution is preferably incubated, namely, kept for a certain period at room temperature or under warming, so as to fix the spotted nucleotide derivatives onto the carrier. In the course of incubation, UV irradiation or surface treatment using sodium borohydride or a Shiff reagent may be applied. The UV irradiation under heating is preferably adopted. It is assumed that these treatments are effective to produce additional linkage or bonding between the solid carrier and the attached oligonucleotide derivatives. The free (namely, unfixed) nucleotide derivatives are washed out using an aqueous solution. Thus washed solid carrier is then dried to give a nucleotide derivative-fixed solid carrier (such as DNA chip) of the invention.

The nucleotide derivative-fixed solid carrier of the invention is favorably employable for monitoring of gene expression, sequencing of base arrangement of DNA, analysis of mutation, analysis of polymorphism, by way of hybridization.

### [Sample Nucleic Acid Fragment -- Target]

A target DID fragment or a sample DNA fragment, which is subjected to the analysis concerning the presence of a complementary DNA fragment can be obtained from various origins. In the analysis of gene, the target DNA fragment is prepared from a cell or tissue of eucaryote. In the analysis of genome, the target DNA fragment is obtained from tissues other than erythrocyte. In the analysis of mRNA, the target sample is obtained from tissues in which mRNA is expressed. If the DNA chip has an oligonucleotide fixed in its solid carrier, the target DNA fragment preferably has a low molecular weight. The target DNA may be multiplied by PCR method.

To the target DNA fragment is attached an RI label or a non-RI label by a known method. The non-RI label is preferably utilized. Examples of the non-RI labels include fluorescence label, biotin label, and chemical luminescence label. The fluorescence label is most preferably employed. Examples of the fluorescence labels include cyanine dyes (e.g., Cy3 and Cy5 belonging to Cy Dye™ series), rhodamine 6G reagent, N-acetoxy-N²-acetylaminofluorene (AAF), and AAIF (iodide derivative of AAF). The target or sample DNA fragments labelled with different fluorescence indicators can be simultaneously analyzed, if the fluorescence indicators have fluorescence spectrum of different peaks. Also employable is an electroconductive label.

### [Hybridization]

The hybridization is performed by spotting an aqueous sample solution containing a target DNA fragment onto a DNA chip. The spotting is generally done in an amount of 1 to 100 nL. The hybridization is carried out by keeping the DNA chip having the spotted sample solution thereon at a temperature between room temperature and 70°C, for 6 to 20 hours. After the hybridization is complete, the DNA chip is washed with an aqueous buffer solution containing a surface active agent, to remove a free (namely, unfixed) sample DNA fragment. The surface active agent preferably is sodium dodecylsulfonate (SDS). The buffer solution may be a citrate buffer solution, a phosphate buffer solution, a borate buffer solution, Tris buffer solution, or Goods buffer solution. The citrate buffer solution is preferably employed.

The present invention is further described by the following examples.

### [Example 1 - Manufacture of Oligonucleotide-fixed plate]

### (1) Preparation of reactive solid carrier

A slide glass (25 mm x 75 mm) was immersed in an ethanol solution of 2 wt.% aminopropyltriethoxysilane (available from Shin-etsu Chemical Industries, Co., Ltd.) for 10 minutes. Subsequently, the slide glass was taken out, washed with ethanol, and dried at 110°C for 10 min. Thus, a silane coupling agent-treated slide glass was prepared.

The silane coupling agent-treated slide glass was then placed in a phosphate buffer solution (pH 8.5) containing 5 wt.% of 1,2-bis(vinylsulfonylacetamide)ethane for one hour. Subsequently, the slide glass was taken out of the solution, washed with acetonitrile, and dried for one hour under reduced pressure, to prepare a reactive plate having a vinylsulfonyl group on its surface.

### (2) Fixation of Oligonucleotide and Measurement of Fluorescence Strength

An oligonucleotide (3'-CTAGTCTGTGAAGTGTCTGATC-5', 22-mers) having L-glutamylglycine at 3'-terminal and a fluorescent label (FluoroLink, Cy5-dCTP, available from Amasham Pharmacia Biotec Corp.) at 5'-terminal was dispersed in 1 µL of an aqueous solution containing a carbonate buffer solution (0.1M, pH 9.3) at a concentration of 1 x 10⁻⁶M. The buffer solution was then spotted onto the reactive plate obtained in (1) above, and this was immediately kept at 25°C, 90%RH for one hour. Thus treated plate was then washed successively twice with a mixture of aqueous 0.1 wt. % SDS (sodium dodecylsulfate) solution and aqueous 2xSSC solution (obtained by twice diluting standard sodium chloride-citrate buffer solution (SSC)), and once with the aqueous 0.2xSSC solution. Thus washed plate was placed in an aqueous 0.1 M glycine solution (pH 10) for 1.5 hours, washed with distilled water, and then dried at room temperature.

The fluorescence strength of thus treated plate was measured using a fluorescence scanning apparatus. The fluorescence strength was 2,500, which was well higher than the background fluorescence strength. This means that the oligonucleotides are well fixed to the slide glass.

### [Example 2 - Detection of Target Oligonucleotide]

### (1) Preparation of DNA chip

An oligonucleotide (3 ' -TCCTCCATGTCCGGGGAGGATCT-GACACTTCAAGGTCTAG-5', 40-mers) having L-glutamylglycine at 3'-terminal and a fluorescent label (FluoroLink, Cy5-dCTP, available from Amasham Pharmacia Biotec Corp.) at 5'-terminal was dispersed in 1 µL of an aqueous solution containing a carbonate buffer solution (0.1 M, pH 9.3) at a concentration of 1 x 10⁻⁶M. The buffer solution was then spotted onto the reactive plate obtained in (1) of Example 1, and this was immediately kept at 25°C, 90%RH for one hour. Thus treated plate was then washed successively twice with a mixture of aqueous 0.1 wt.% SDS (sodium dodecylsulfate) solution and aqueous 2xSSC solution (obtained by twice diluting standard sodium chloride-citrate buffer solution (SSC)), and once with the aqueous 0.2xSSC solution. Thus washed plate was placed in an aqueous 0.1 M glycine solution (pH 10) for 1.5 hours, washed with distilled water, and then dried at room temperature, to prepare a DNA chip.

### (2) Detection of target oligonucleotide

A target oligonucleotide (CTAGTCTGTGAAGTTCCAGATC-5 , 22-mers) having Cy5 (fluorescent label) at its 5'-terminal was dispersed in 20 µL of a hybridizing solution (mixture of 4 x SSC and 10 wt.% SDS) . The resulting solution was spotted onto the DNA chip prepared in (1) above, and its spotted surface was covered with a covering glass. Thus covered chip was subjected to incubation at 60°C for 20 hours in a moisture chamber. The incubated chip was washed successively with a mixture of O.1 wt.% SDS and 2xSSC, a mixture of 0.1 wt.% SDS and 0.2xSSC, and an aqueous 0.2xSSC solution, centrifuged at 600 r-p.m. for 20 seconds, and dried at room temperature.

The fluorescence strength of thus treated DNA chip was measured using a fluorescence scanning apparatus. The fluorescence strength was 1,219, which was well higher than the background fluorescence strength. This means that the target oligonucleotides are well fixed to the DNA chip having the complementary oligonucleotide probe.

### [Example 3 - Manufacture of Oligonucleotide-fixed plate]

### (1) Preparation of reactive solid carrier

A slide glass (25 mm x 75 mm) was immersed in an ethanol solution of 2 wt.% aminopropyltriethoxysilane (available from Shin-etsu Chemical Industries, Co., Ltd.) for 10 minutes. Subsequently, the slide glass was taken out, washed with ethanol, and dried at 110°C for 10 min. Thus, a silane coupling agent-treated slide glass was prepared.

The silane coupling agent-treated slide glass was then placed in a solution of 0.5 g of chlorosulfonyl isocyanate in 1 mL of acetonitrile for 2 hours, taken out of the acetonitrile solution, washed with acetonitrile, and dried for one hour under reduced pressure, to prepare a reactive plate.

### (2) Fixation of Oligonucleotide and Measurement of Fluorescence Strength

An oligonucleotide (3'-CTAGTCTGTGAAGTGTCTGATC-5 , 22-mers) having L-glutamylglycine at 3'-terminal and a fluorescent label (FluoroLink, Cy5-dCTP, available from Amasham Pharmacia Biotec Corp.) at 5'-terminal was dispersed in 1 µL of an aqueous solution containing a carbonate buffer solution (0.1 M, pH 8.0) at a concentration of 1 x 10⁻⁶M. The buffer solution was then spotted onto the reactive plate obtained in (1) above, and this was immediately kept at 25°C, 90%RH for one hour. Thus treated plate was then washed successively twice with a mixture of aqueous 0.1 wt.% SDS solution and aqueous 2xSSC solution, and once with the aqueous 0.2xSSC solution. Thus washed plate was placed in an aqueous 0.1 M glycine solution (pH 10) for 1.5 hours, washed with distilled water, and then dried at room temperature.

The fluorescence strength of thus treated plate was measured using a fluorescence scanning apparatus. The fluorescence strength was 3,100, which was well higher than the background fluorescence strength. This means that the oligonucleotides are well fixed to the slide glass.

### [Example 4 - Detection of Target Oligonucleotide]

### (1) Preparation of DNA chip

The procedure of Example 1-(1) was repeated except for using the same nucleotide oligomer but having no fluorescent label at 3' -terminal, to prepare a DNA chip.

### (2) Detection of target oligonucleotide

A target oligonucleotide (CTAGTCTGTGAAGTTCCAGATC-5', 22-mers) having Cy5 (fluorescent label) at its 5'-terminal was dispersed in 20 µL of a hybridizing solution (mixture of 4 x SSC and 10 wt.% SDS) . The resulting solution was spotted onto the DNA chip prepared in (1) above, and its spotted surface was covered with a covering glass. Thus covered chip was subjected to incubation at 60°C for 20 hours in a moisture chamber. The incubated chip was washed successively with a mixture of 0.1 wt,% SDS and 2xSSC, a mixture of 0.1 wt.% SDS and 0.2xSSC, and an aqueous 0.2xSSC solution, centrifuged at 600 r.p.m. for 20 seconds, and dried at room temperature.

The fluorescence strength of thus treated DNA chip was measured using a fluorescence scanning apparatus. The fluorescence strength was 1,078, which was well higher than the background fluorescence strength. This means that the target oligonucleotides are well fixed to the DNA chip having the complementary oligonucleotide probe.

### [Example 5 - Manufacture of Oligonucleotide-fixed plate]

The procedures of Example 3-(1) and-(2) were repeated except for employing 0.5 g of succinimidinyl (4-vinylsulfonyl)benzoate in place of the chlorosulfonyl isocyanate, to prepare an oligonucleotide-fixed plate.

The fluorescence strength of the prepared oligonucleotide-fixed plate was measured using a fluorescence scanning apparatus. The fluorescence strength was 3,250, which was well higher than the background fluorescence strength. This means that the oligonucleotides are well fixed to the slide glass.

### [Example 6 - Detection of Target Oligonucleotide]

### (1) Preparation of DNA chip

The procedures of Example 3 were repeated except for using the same nucleotide oligomer but having no fluorescent label at 3'-terminal, to prepare a DNA chip.

### (2) Detection of target oligonucleotide

The procedures of detection of target oligonucleotide were repeated using the DNA chip obtained in (1) above..

The fluorescence strength of thus treated DNA chip was measured using a fluorescence scanning apparatus. The fluorescence strength was 2,325, which was well higher than the background fluorescence strength. This means that the target oligonucleotides are well fixed to the DNA chip having the complementary oligonucleotide probe.

### [Example 7 - Detection of Target Oligonucleotide]

### (1) Synthesis of complex of vinylsulfone compound and silane-coupling agent

Vinylsulfonylacetoacetic acid (1.5 g) was dissolved in 75 mL of tetrahydrofuran. To the resulting solution were added successively 2.2 g of 3-aminopropyltriethoxysilane and 2.1 g of N,N-dicyclohexylcarbodiimide (DCC, condensating reagent). The mixture was stirred for 2 hours at room temperature The reaction mixture was concentrated and purified by silica gel column chromatography (eluent: hexane/ethyl acetate = 10/1) to give the desired complex.

### (2) Production of divinylsulfone compound-fixed plate

The complex obtained above was dissolved in water to give an aqueous 2% solution. In the solution was placed a slide glass (25 mm x 75 mm) for 10 minutes. The slide glass was taken out, washed with ethanol, and dried at 110°C for 10 minutes, to give the desired vinylsulfone compound-fixed plate, that is, a reactive plate.

### (3) Fixation of Oligonucleotide and Measurement of Fluorescence Strength

An oligonucleotide (3 ' -CTAGTCTGTGAAGTGTCTGATC-5 ', 22-mers) having L-glutamylglycine at 3'-terminal and a fluorescent label (FluoroLink, Cy5-dCTP, available from Amasham Pharmacia Biotec Corp.) at 5'-terminal was dispersed in 1 µL of an aqueous solution containing a carbonate buffer solution (0.1 M, pH 8.0) at a concentration of 1 x 10⁻⁶M. The buffer solution was then spotted onto the reactive plate obtained in (2) above, and this was immediately kept at 25°C, 90%RH for one hour. Thus treated plate was then washed successively twice with a mixture of aqueous 0.1 wt.% SDS solution and aqueous 2xSSC solution, and once with the aqueous 0.2xSSC solution. Thus washed plate was placed in an aqueous 0.1 M glycine solution (pH 10) for 1.5 hours, washed with distilled water, and then dried at room temperature.

The fluorescence strength of thus treated plate was measured using a fluorescence scanning apparatus. The fluorescence strength was 3,500, which was well higher than the background fluorescence strength. This means that the oligonucleotides are well fixed to the slide glass.

### [Example 8 - Manufacture of Oligonucleotide-fixed electrode]

### (1) Preparation of reactive electrode

On a gold electrode (surface area: 2.25 mm²) was spotted 2 µL of an aqueous 1 mM 11-amino-1-undecathiol solution. The spotted solution was allowed to stand for 10 hours at room temperature, keeping the solution from evaporating, and then washed successively with distilled water and ethanol. On thus treated gold electrode was spotted a phosphate buffer (pH 8.5) containing 3% of 1,2-bis (vinylsulfonylacetamide)ethane. The spotted electrode was allowed to stand for 2 hours at room temperature, washed successively with distilled water and ethanol, and dried for one hour under reduced pressure, to give a gold electrode having a vinylsulfonyl group which was covalently attached to the surface of the electrode, that is, a reactive electrode.

### (2) Fixation of oligonucleotide

On the reactive electrode was spotted 2 µl of an aqueous solution containing 100 picomol./µL of T₂₀ (thymine 20-mers having an aminohexyl group at its 5'-terminal). The spotted solution was allowed to stand for one hour at room temperature, and the unfixed T₂₀ was washed out, and dried, to give an electrochemical analytical element.

### (3) Preparation of ferocene-labeled oligonucleotide

An aminohexyl linker was connected to the 5'-terminal of adenine 20-mers (A₂₀). Thus prepared oligonucleotide having the aminohexyl linker was treated in the manner described in Takenaka et al, Analytical Biochemistry, 218, 436-443 (1994), to obtain an adenine 20-mers which was labeled with a ferocene group at its 5'-terminal (F1-A₂₀)

### (4) Detection of complementary target oligonucleotide

On the analytical element prepared in (2) above was spotted 2 µL of a Tris buffer (10 mM, pH 7.5) containing the F1-A₂₀ obtained in (3) above. The analytical element was then kept at 25°C for 30 minutes for performing incubation. The incubated element was washed with pure water to remove the unfixed F1-A₂₀.

Thus treated element was placed in 0.1 M potassium chloride-0.1 M acetic acid buffer (pH 5.60) and subjected to differential pulse voltammetry (DVP) in the applied voltage range of 100 to 700 mV. A responsive electric current at 460 mV corresponding to F1-A₂₀ was detected.

### [Example 9 - Manufacture of Oligonucleotide- fixed electrode -- illustrated in Fig. 3]

### (1) Preparation of reactive solid carrier

A slide glass (25 mm x 75 mm) was immersed in an ethanol solution of 2 wt-% aminopropyltriethoxysilane (available from Shin-etsu Chemical Industries, Co., Ltd.) for 10 minutes. Subsequently, the slide glass was taken out, washed with ethanol, and dried at 110°C for 10 min. Thus, a silane coupling agent-treated slide glass was prepared.

The silane coupling agent-treated slide glass was then placed for one hour in a phosphate buffer solution (pH 8.5) containing 5 wt.% of a copolymer having the following formula:

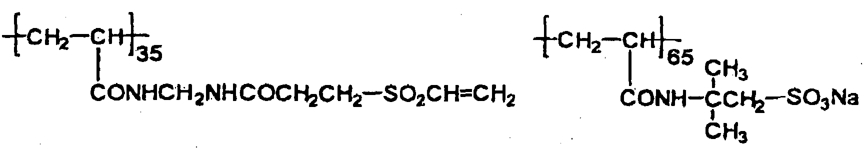

The slide glass was taken out of the buffer solution, washed with acetonitrile, and dried for one hour under reduced pressure, to prepare a reactive plate.

### (2) Fixation of Oligonucleotide and Measurement of Fluorescence Strength

An oligonucleotide (3'-CTAGTCTGTGAAGTGTCTGATC-5', 22-mers) having L-glutamylglycine at 3'-terminal and a fluorescent label (FluoroLink, Cy5-dCTP, available from Amasham Pharmacia Biotec Corp.) at 5'-terminal was dispersed in 1 µL of an aqueous solution containing a carbonate buffer solution (0.1 M, pH 9.3) at a concentration of 1 x 10⁻⁶M. The buffer solution was then spotted onto the reactive plate obtained in (1) above, and this was immediately kept at 25°C, 90%RH for one hour. Thus treated plate was then washed successively twice with a mixture of aqueous 0.1 wt.% SDS solution and aqueous 2xSSC solution, and once with the aqueous 0.2xSSC solution. Thus washed plate was placed in an aqueous 0.1 M glycine solution (pH 10) for 1.5 hours, washed with distilled water, and then dried at room temperature.

The fluorescence strength of thus treated plate was measured using a fluorescence scanning apparatus. The fluorescence strength was 3,100, which was well higher than the background fluorescence strength. This means that the oligonucleotides are well fixed to the slide glass.

### [Example 10 - Manufacture of Oligonucleotide-fixed electrode -- illustrated in Fig. 4]

### (1) Preparation of reactive solid carrier

A slide glass (25 mm x 75 mm) was immersed in an aqueous solution of 2 wt.% aminopropyltriethoxysilane (available from Shin-etsu Chemical Industries, Co., Ltd.) for 10 minutes. Subsequently, the slide glass was taken out, washed with ethanol, and dried at 110°C for 10 min. Thus, a silane coupling agent-treated slide glass was prepared.

The silane coupling agent-treated slide glass was placed in a borate buffer solution (pH 8.0) containing 3 wt.% of 1,2-bis(vinylsulfonylacetamide) ethane for 2 hours. Subsequently, the slide glass was taken out of the solution, washed with water, and dried for one hour under reduced pressure, to prepare a reactive plate having a vinylsulfonyl group on its surface.

Thus prepared reactive plate was placed in a borate buffer solution (pH 8.0) containing 2 wt.% of polyarylamine (M.W. 10,000, available from Nittobo Co., Ltd.) for 2 hours. Subsequently, the slide glass was taken out of the solution, washed with water, and dried for one hour under reduced pressure, to prepare a surface amine-group increased plate.

The surface amine-group increased plate was then placed in a phosphate buffer solution (pH 8.0) containing 3 wt.% of 1,2-bis(vinylsulfonylacetamide)ethane for 2 hours. Subsequently, the plate was taken out of the solution, washed with water, and dried for one hour under reduced pressure, to prepare a reactive plate having an increased number of vinylsulfonyl groups on its surface.

## Claims

1. A reactive solid carrier comprising a solid carrier and a plurality of vinylsulfonyl groups or their reactive precursors each of which is fixed onto a surface of the solid carrier by covalent bonding via a linking group.

2. The reactive solid carrier of claim 1, wherein the vinylsulfonyl group or its reactive precursor linked to the linking group is represented by the following formula:
- L - SO₂ - X
in which L represents a linking group, and X represents a group of -CR¹=CR²R³ or -CHR¹-CR²R³Y wherein each of R¹, R² and R³ independently is a hydrogen atom, an alkyl group having 1 to 6 carbon atoms, an aryl group having 6 to 20 carbon atoms, or an aralkyl group having 7 to 26 carbon atoms in which its alkyl group has 1 to 6 carbon atoms; Y represents a halogen atom, -SO₂R¹¹, -OCOR¹², -OSO₃M, or a quaternary pyridinium group; R¹¹ is a hydrogen atom, an alkyl group having 1 to 6 carbon atoms, an aryl group having 6 to 20 carbon atoms, or an aralkyl group having 7 to 26 carbon atoms in which its alkyl group has 1 to 6 carbon atoms; R¹² is an alkyl group having 1 to 6 carbon atoms, or a halogenated alkyl group having 1 to 6 carbon atoms; and M is a hydrogen atom, an alkali metal atom, or an ammonium group.

3. The reactive solid carrier of claim 2, wherein X is a vinyl group having the formula of -CH=CH₂.

4. The reactive solid carrier of claim 2, wherein L is a linking group having an atom of divalence or multiple valence other than a carbon atom.

5. The reactive solid carrier of claim 2, wherein L has a linking atom represented by -NH-, -S-, or -O-.

6. The reactive solid carrier of claim 2, wherein L is a linking group represented by -(L¹)ₙ-NH-(CR¹CR²)₂- or -(L¹)ₙ-S-(CR¹CR²)₂-, in which each of R¹ and R² has the meaning as defined in claim 2, and L¹ is a linking group and n is 0 or 1.

7. The reactive solid carrier of claim 2, wherein L is a linking group represented by -(L¹)n-NHCH₂CH₂-, in which L¹ is a linking group and n is 0 or 1.

8. The reactive solid carrier of claim 7, wherein which L¹ has a linking group of -SiO- and n is 0 or 1.

9. The reactive solid carrier of claim 1, wherein the solid carrier is a glass plate, a resin plate, a glass or resin plate which has been treated with a silane coupling agent, or a glass or resin plate having a coating layer thereon.

10. The reactive solid carrier of claim 9, wherein the solid carrier is a silicate glass plate, a silicate glass plate having been treated with a silane coupling agent, or a silicate glass plate having an organic material coat.

11. A method for preparing the reactive solid carrier of claim 2, which comprises bringing a solid carrier having reactive groups on its surface into contact with disulfone compounds having the following formula:
X¹ - SO₂ - L² - SO₂ - X²
in which L² represents a linking group, and each of X¹ and X² represents a group of -CR¹=CR²R³ or -CHR¹-CR²R³Y wherein each of R¹, R² and R³ independently is a hydrogen atom, an alkyl group having 1 to 6 carbon atoms, an aryl group having 6 to 20 carbon atoms, or an aralkyl group having 7 to 26 carbon atoms in which its alkyl group has 1 to 6 carbon atoms; Y represents a halogen atom, -SO₂R¹¹, -OCOR¹², -OSO₃M, or a quaternary pyridinium group; R¹¹ is a hydrogen atom, an alkyl group having 1 to 6 carbon atoms, an aryl group having 6 to 20 carbon atoms, or an aralkyl group having 7 to 26 carbon atoms in which its alkyl group has 1 to 6 carbon atoms; R¹² is an alkyl group having 1 to 6 carbon atoms, or a halogenated alkyl group having 1 to 6 carbon atoms; and M is a hydrogen atom, an alkali metal atom, or an ammonium group.

12. The method of claim 11, wherein the reactive groups are amino groups, mercapto groups or hydroxyl groups.

13. A method for producing a solid carrier having nucleotide derivatives or their analogues fixed onto its surface, which comprises bringing a reactive solid carrier comprising a solid carrier and a plurality of vinylsulfonyl groups or their reactive precursors each of which is fixed onto a surface of the solid carrier by covalent bonding into contact with nucleotide derivatives or their analogues having a reactive group which is reactive with the vinylsulfonyl group or reactive precursor.

14. The method of claim 13, wherein the nucleotide derivatives or the analogues are oligonucleotides, polynucleotides, or peptide nucleic acids.

15. The method of claim 13, wherein the vinylsulfonyl group or its reactive precursor linked to the linking group is represented by the following formula:
- L - SO₂ - X
in which L represents a linking group, and X represents a group of -CR¹=CR²R³ or -CHR¹-CR²R³Y wherein each of R¹, R² and R³ independently is a hydrogen atom, an alkyl group having 1 to 6 carbon atoms, an aryl group having 6 to 20 carbon atoms, or an aralkyl group having 7 to 26 carbon atoms in which its alkyl group has 1 to 6 carbon atoms; Y represents a halogen atom, -SO₂R¹¹, -OCOR¹², -OSO₃M, or a quaternary pyridinium group; R11 is a hydrogen atom, an alkyl group having 1 to 6 carbon atoms, an aryl group having 6 to 20 carbon atoms, or an aralkyl group having 7 to 26 carbon atoms in which its alkyl group has 1 to 6 carbon atoms; R¹² is an alkyl group having 1 to 6 carbon atoms, or a halogenated alkyl group having 1 to 6 carbon atoms; and M is a hydrogen atom, an alkali metal atom, or an ammonium group.

16. The method of claim 15, wherein X is a reactive group having the formula of -CR¹=CR²R³ in which each of R¹, R² and R³ has the meaning as defined in claim 15.

17. A solid carrier having nucleotide derivatives or their analogues fixed onto its surface which is produced by a method of claim 13.

18. A method comprising bringing a solid carrier of 17 having nucleotide derivatives or their analogues fixed onto its surface into contact with. oligonucleotides or polynucleotides which are complementary to the nucleotide derivatives or their analogues fixed onto the surface of the solid carrier in the presence of an aqueous solvent, so as to combine the complementary oligonucleotides or polynucleotides with the nucleotide derivatives or their analogues.

19. The method of claim 18, wherein the oligonucleotides or polynucleotides have a detectable label.

20. A combination of a solid carrier of claim 17 having nucleotide derivatives or their analogues fixed onto its surface and oligonucleotides or polynucleotides complementary to the nucleotide derivatives or their analogues, in which the oligonucleotides or polynucleotides are combined complementarily with the nucleotide derivatives or their analogues.

21. The combination of claim 20, wherein the oligonucleotides or polynucleotides have a detectable label.

22. A method for preparing the reactive solid carrier of claim 2, which comprises bringing a solid carrier having reactive groups on its surface into contact with bifunctional compounds having the following formula:
X¹ - SO₂ - L² - X³
in which L² represents a linking group, X¹ represents a group of -CR¹=CR²R³ or -CHR¹-CR²R³Y wherein each of R¹, R² and R³ independently is a hydrogen atom, an alkyl group having 1 to 6 carbon atoms, an aryl group having 6 to 20 carbon atoms, or an aralkyl group having 7 to 26 carbon atoms in which its alkyl group has 1 to 6 carbon atoms; Y represents a halogen atom, -SO₂R¹¹, -OCOR¹², -OSO₃M, or a quaternary pyridinium group; R¹¹ is a hydrogen atom, an alkyl group having 1 to 6 carbon atoms, an aryl group having 6 to 20 carbon atoms, or an aralkyl group having 7 to 26 carbon atoms in which its alkyl group has 1 to 6 carbon atoms; R¹² is an alkyl group having 1 to 6 carbon atoms, or a halogenated alkyl group having 1 to 6 carbon atoms; and M is a hydrogen atom, an alkali metal atom, or an ammonium group, and X³ represents a maleimide group, a halogen atom, an isocyanate group, a thioisocyanate group, a succimidoxy group, an aldehyde group, or a carboxyl group.

23. A reactive solid carrier comprising a solid carrier and a plurality of vinylsulfonyl groups or their reactive precursors each of which is fixed onto a surface of the solid carrier by covalent bonding via hydrophilic polymer chains.

24. The reactive solid carrier of claim 23, wherein the vinylsulfonyl group or its reactive precursor linked to the linking group is represented by the following formula:
- L - SO₂ - X
in which L represents a hydrophilic polymer chain, and X represents a group of -CR¹=CR²R³ or -CHR¹-CR²R³Y wherein each of R¹, R² and R³ independently is a hydrogen atom, an alkyl group having 1 to 6 carbon atoms, an aryl group having 6 to 20 carbon atoms, or an aralkyl group having 7 to 26 carbon atoms in which its alkyl group has 1 to 6 carbon atoms; Y represents a halogen atom, -SO₂R¹¹, -OCOR¹², -OSO₃M, or a quaternary pyridinium group; R¹¹ is a hydrogen atom, an alkyl group having 1 to 6 carbon atoms, an aryl group having 6 to 20 carbon atoms, or an aralkyl group having 7 to 26 carbon atoms in which its alkyl group has 1 to 6 carbon atoms; R¹² is an alkyl group having 1 to 6 carbon atoms, or a halogenated alkyl group having 1 to 6 carbon atoms; and M is a hydrogen atom, an alkali metal atom, or an ammonium group.

25. A method for producing a solid carrier having nucleotide derivatives or their analogues fixed onto its surface, which comprises bringing a reactive solid carrier comprising a solid carrier and a plurality of vinylsulfonyl groups or their reactive precursors each of which is fixed onto a surface of the solid carrier by covalent bonding via a hydrophilic polymer chain into contact with nucleotide derivatives or their analogues having a reactive group which is reactive with the vinylsulfonyl group or reactive precursor.

26. The method of claim 25, wherein the nucleotide derivatives or the analogues are oligonucleotides, polynucleotides, or peptide nucleic acids.

27. The method of claim 25, wherein the vinylsulfonyl group or its reactive precursor linked to the linking group is represented by the following formula:
- L - SO₂ - X
in which L represents a hydrophilic polymer chain group, and X represents a group of -CR¹=CR²R³ or -CHR¹-CR²R³Y wherein each of R¹, R² and R³ independently is a hydrogen atom, an alkyl group having 1 to 6 carbon atoms, an aryl group having 6 to 20 carbon atoms, or an aralkyl group having 7 to 26 carbon atoms in which its alkyl group has 1 to 6 carbon atoms; Y represents a halogen atom, -SO₂R¹¹, -OCOR¹², -OSO₃M, or a quaternary pyridinium group; R¹¹ is a hydrogen atom, an alkyl group having 1 to 6 carbon atoms, an aryl group having 6 to 20 carbon atoms, or an aralkyl group having 7 to 26 carbon atoms in which its alkyl group has 1 to 6 carbon atoms; R¹² is an alkyl group having 1 to 6 carbon atoms, or a halogenated alkyl group having 1 to 6 carbon atoms; and M is a hydrogen atom, an alkali metal atom, or an ammonium group.

28. The method of claim 27, wherein X is a reactive group having the formula of -CR¹=CR²R³ in which each of R1, R² and R³ has the meaning as defined in claim 27.

29. A solid carrier having nucleotide derivatives or their analogues fixed onto its surface which is produced by a method of claim 25.

30. A reactive solid carrier comprising a solid carrier and a plurality of vinylsulfonyl groups or their reactive precursors a number of which are linked to a polymer chain which is fixed onto a surface of the solid carrier by covalent bonding at a number of sites which number is smaller than the number of vinylsulfonyl groups or their reactive precursors linked to the polymer chain.

31. The reactive solid carrier of claim 30, wherein the vinylsulfonyl group or its reactive precursor linked to the polymer chain is represented by the following formula:
- L -(- SO₂ - X)ₖ
in which L represents a polymer chain, X represents a group of -CR¹=CR²R³ or -CHR¹-CR²R³Y wherein each of R¹, R² and R³ independently is a hydrogen atom, an alkyl group having 1 to 6 carbon atoms, an aryl group having 6 to 20 carbon atoms, or an aralkyl group having 7 to 26 carbon atoms in which its alkyl group has 1 to 6 carbon atoms ; Y represents a halogen atom, -SO₂R¹¹, -OCOR¹², -OSO₃M, or a quaternary pyridinium group; R¹¹ is a hydrogen atom, an alkyl group having 1 to 6 carbon atoms, an aryl group having 6 to 20 carbon atoms, or an aralkyl group having 7 to 26 carbon atoms in which its alkyl group has 1 to 6 carbon atoms; R¹² is an alkyl group having 1 to 6 carbon atoms, or a halogenated alkyl group having 1 to 6 carbon atoms; and M is a hydrogen atom, an alkali metal atom, or an ammonium group, and k is an integer of 2 or more.

32. A method for producing a solid carrier having nucleotide derivatives or their analogues fixed onto its surface, which comprises bringing a reactive solid carrier comprising a solid carrier and a plurality of vinylsulfonyl groups or their reactive precursors a number of which are linked to a polymer chain which is fixed onto a surface of the solid carrier by covalent bonding at a number of sites which number is smaller than the number of vinylsulfonyl groups or their reactive precursors linked to the polymer chain into contact with nucleotide derivatives or their analogues having a reactive group which is reactive with the vinylsulfonyl group or reactive precursor.

33. The method of claim 32, wherein the nucleotide derivatives or the analogues are oligonucleotides, polynucleotides, or peptide nucleic acids.

34. The method of claim 32, wherein the vinylsulfonyl group or its reactive precursor linked to the polymer chain is represented by the following formula:
- L -(- SO₂ - X)ₖ
in which L represents a polymer chain, X represents a group of -CR¹=CR²R³ or -CHR¹-CR²R³Y wherein each of R¹, R² and R³ independently is a hydrogen atom, an alkyl group having 1 to 6 carbon atoms, an aryl group having 6 to 20 carbon atoms, or an aralkyl group having 7 to 26 carbon atoms in which its alkyl group has 1 to 6 carbon atoms; Y represents a halogen atom, -SO₂R¹¹, -OCOR¹², -OSO₃M, or a quaternary pyridinium group; R¹¹ is a hydrogen atom, an alkyl group having 1 to 6 carbon atoms, an aryl group having 6 to 20 carbon atoms, or an aralkyl group having 7 to 26 carbon atoms in which its alkyl group has 1 to 6 carbon atoms; R¹² is an alkyl group having 1 to 6 carbon atoms, or a halogenated alkyl group having 1 to 6 carbon atoms; and M is a hydrogen atom, an alkali metal atom, or an ammonium group, and k is an integer of 2 or more.

35. The method of claim 34, wherein X is a reactive group having the formula of -CR¹=CR²R³ in which each of R¹, R² and R³ has the meaning as defined in claim 34.

36. A solid carrier having nucleotide derivatives or their analogues fixed onto its surface which is produced by a method of claim 32.


### Annex to application documents - subsequently filed sequences listing

SEQUENCE LISTING

<110> FUJI PHOTO FILM CO., LTD.

<120> DNA CHIP AND REACTIVE SOLID CARRIER

<130> 11263

<140> EP 00 126 448.0
<141> 2000-12-06

<150> JP 11-346157
<151> 1999-12-06

<150> JP 11-363795
<151> 1999-12-22

<150> JP 2000-084373
<151> 2000-03-24

<160> 3

<170> PatentIn Ver. 2.1

<210> 1
<211> 22
<212> DNA
<213> Artificial Sequence

<220>
<223> Description of Artificial Sequence:
      Oligonucleotide

<400> 1
ctagtctgtg aagtgtctga tc                                          22

<210> 2
<211> 23
<212> DNA
<213> Artificial Sequence

<220>
<223> Description of Artificial Sequence:
      Oligonucleotide

<400> 2
tcctccatgt ccggggagga tct                                         23

<210> 3
<211> 22
<212> DNA
<213> Artificial Sequence

<220>
<223> Description of Artificial Sequence: Target
      oligonucleotide

<400> 3
ctagtctgtg aagttccaga tc                                                22